# EUROPÄISCHE PATENTANMELDUNG

(11) **EP 1 902 690 A1**
(43) Veröffentlichungstag der Anmeldung: **26.03.2008**
(21) Anmeldenummer: 07012560.4
(22) Anmeldetag: 27.06.2007
(51) Int. Cl.: A61F 2/44

(54) **Bandscheibenprothese**

(30) Priorität: 21.09.2006 DE 102006045108
(71) Anmelder: Fehling AG, 4153 Reinach BL 1 (CH)
(72) Erfinder: Fehling, Guido, 63791 Karlstein (DE); Fehling, Gerald, 63791 Karlstein (DE)
(74) Vertreter: Patentanwälte Westphal, Mussgnug & Partner

(57) **Zusammenfassung**

Eine implantierbare Bandscheibenprothese besteht aus einer kranialen Endplatte (10) und einer kaudalen Endplatte (12), zwischen denen Federmittel angeordnet sind. Die Federmittel bestehen aus einer Memory-Metalllegierung, die bei Körpertemperatur superelastische Eigenschaften aufweist, Die Federmittel sind als zu den Endplatten (10, 12) flächenparallele Flächengebilde (14) ausgebildet, die aus in der Ebene dieser Flächengebilde (14) verlaufenden gespannten Drähten aus der Memory-Metalllegierung bestehen.

## Beschreibung

Die Erfindung betrifft eine Bandscheibenprothese gemäß dem Oberbegriff des Patentanspruchs 1.

Die Bandscheiben dienen als druckelastische Abstützung zwischen den Wirbelkörpern der Wirbelsäule. Schäden der Bandscheiben, insbesondere durch Degeneration und Verschleiß können zu schweren Einschränkungen der Beweglichkeit und neurologischen Symptomen, insbesondere Schmerzen und Lähmungen führen. Sind solche Erkrankungen nicht mehr konservativ zu behandeln, so ist es bekannt, die defekte Bandscheibe durch eine implantierte Bandscheibenprothese zu ersetzen. Eine solche Bandscheibenprothese besteht aus einer oberen kranialen Endplatte und einer unteren kaudalen Endplatte, zwischen welchen Federmittel eingesetzt sind, die diese Endplatten druckelastisch gegeneinander abstützen. Die Bandscheibenprothese wird anstelle der entfernten Bandscheibe zwischen die Wirbel implantiert, wobei sie mit den Endplatten an den Wirbelkörpern des darüberliegenden bzw. des darunterliegenden Wirbels verankert werden.

Aus der EP 1 273 276 B1 ist es bekannt, Federmittel aus einer Memory-Metalllegierung, vorzugsweise aus einer Nickel-TitanLegierung zu verwenden, die bei Körpertemperatur superelastische Eigenschaften aufweist. Die Superelastizität, auch Pseudoelastizität genannt, beruht auf speziellen Gefügeeigeschaften der Austenit-Martensit-Umwandlung in einem relativ engen Temperaturbereich. In diesem Temperaturbereich bildet sich bei mechanischer Beanspruchung aus dem Austenit ein spannungsinduzierter Martensit, der ein elastisches Verhalten zur Folge hat. Nach der Entlastung bildet sich das Austenitgefüge wieder zurück und das Bauelement kehrt in seinen Ausgangszustand zurück. Im Bereich der Superelastizität können Verformungen bis zu 8 % bei nahezu konstanter Spannung erhalten werden, so dass Federmittel hergestellt werden können, die unabhängig von ihrer Verformung eine kontinuierliche Kraft ausüben.

In der EP 1 273 276 B1 sind verschiedene Ausführungen für diese aus der Memory-Metalllegierung bestehenden Federmittel beschrieben. In einer Ausführung sind die Federmittel durch eine achsmittig zwischen den Endplatten angeordnete Schraubendruckfeder gebildet. Eine solche Schraubendruckfeder ist einfach herzustellen und weist bei ihrer axialen Verformung nur geringe Materialdehnungen auf. Allerdings sind die erzielbaren Federkräfte begrenzt. In einer anderen Ausführung wird eine Schraubenfeder verwendet, die in Form eines Torus ringförmig zwischen den Endscheiben angeordnet ist. Hierdurch können größere Federkräfte erhalten werden, der verwendete Federdraht wird jedoch stark auf Biegung beansprucht. In einer weiteren Ausführung werden als Federmittel Tellerfedern verwendet. Hierbei können hohe Federkräfte bei geringer Bauhöhe erzielt werden und die Federelemente sind einfach in der Herstellung. Zwischen den einzelnen Federscheiben treten jedoch bei der Verformung Reibungseffekte auf und die bei der Wirbeldrehung auftretenden Rotationsbewegungen können nicht abgefedert werden. Schließlich sind auch Federmittel beschrieben, die durch eine oder mehrere gewölbte Blattfedern gebildet sind. Diese Federn werden ebenfalls sehr stark auf Biegung beansprucht.

Der Erfindung liegt daher die Aufgabe zu Grunde, eine Bandscheibenprothese der eingangs genannten Gattung zur Verfügung zu stellen, bei welcher die Federmittel bei geringer axialer Bauhöhe hohe axiale Belastungen mit einer geringen Materialbeanspruchung aufnehmen können.

Diese Aufgabe wird erfindungsgemäß gelöst durch eine Bandscheibenprothese mit den Merkmalen des Patentanspruchs 1.

Vorteilhafte Ausführungen der Erfindung sind in den Unteransprüchen angegeben.

Der wesentliche Gedanke der Erfindung besteht darin, die Federmittel mit wenigstens einem Flächengebilde auszubilden, welches im Wesentlichen parallel zu den Endplatten verläuft und aus in der Ebene des Flächengebildes gespannten Drähten der Memory-Metalllegierung besteht. Dieses Flächengebilde ist mit seinem zentrischen Mittenbereich mit einer der Endplatten und mit seinem radial beabstandeten äußeren Umfangsbereich mit der anderen Endplatte verbunden. Eine axiale Bewegung der beiden Endplatten gegeneinander führt somit dazu, dass sich der Mittenbereich und der Umfangsbereich des Flächengebildes axial gegeneinander bewegen. Diese axiale Relativbewegung des Mittenbereichs und des Umfangsbereichs führen bei dem radialen Abstand zwischen Mittenbereich und Umfangsbereich und den üblichen axialen Hubbewegungen der Endplatte im Wesentlichen zu einer Längsdehnung der Drähte und nur zu einer geringen Verbiegung der Drähte, so dass die Materialdehnung insgesamt und auch auf der Außenseite der Biegung gering bleibt. Da die Federmittel im Wesentlichen nur die Ebene der Flächengebilde beanspruchen, haben die Federmittel eine äußerst geringe axiale Bauhöhe und beeinflussen die anatomische Gestaltung der implantierbaren Bandscheibenprothese nicht. Die vertikale Belastung der Bandscheibenprothese wird in eine horizontale Dehnung der Drähte des Flächengebildes umgeleitet. Dadurch wird das Verhältnis der Länge der Drähte zu deren Dehnungsweg optimiert. Bei hohen axialen Reaktionskräften treten nur geringe Materialdehnungen auf, so dass sich die Federmittel durch eine hohe Dauerstandfestigkeit auszeichnen.

Grundsätzlich ist es erfindungsgemäß möglich, nur ein Flächengebilde als Federmittel zu verwenden, das mit seinem Umfangsbereich an der einen Endplatte und mit seinem Mittenbereich an der anderen Endplatte festgelegt ist. Einen größeren axialen Hubweg erhält man in einer bevorzugten Ausführung dadurch, dass zwei Flächengebilde verwendet werden, die jeweils mit einer der Endplatten verbunden sind und über einen zwischen den Flächengebilden angeordneten Kern verbunden und damit gewissermaßen in Reihe geschaltet sind. Der Mittenbereich des Flächengebildes der einen Endplatte ist somit über den Kern und das zweite Flächengebilde mit der anderen Endplatte verbunden. Zweckmäßig sind dabei die beiden Flächengebilde jeweils mit ihrem Umfangsbereich mit der zugehörigen Endplatte verbunden, während die Mittenbereiche der beiden Flächengebildet durch den dazwischen angeordneten Kern miteinander verbunden sind.

Sind die Flächengebilde mit ihrem Umfangsbereich an der Endplatte angebracht, so weist diese Endplatte vorzugsweise eine koaxiale konkave Einwölbung auf, in welche das Flächegebilde mit seinem Mittenbereich bei einer axialen Belastung hineingedrückt werden kann. Dadurch ergibt sich eine besonders günstige axiale Bauhöhe. Der die Flächengebilde verbindende Kern, der bei einer axialen Belastung das Flächengebilde in die Einwölbung der Endplatte hineindrückt, ist vorzugsweise koaxial konvex ausgewölbt, so dass sich die Drähte des Flächengebildes bei einer Auslenkung und axialen Belastung an die konvexe Wölbung des Kerns anlegen und damit eine gleichmäßige Verteilung der Biegung und damit der Dehnungsbeanspruchung über die gesamte Länge der Drähte gewährleistet wird.

In einer Ausführung ist das Flächengebilde aus sich netzartig kreuzend gespannten Drähten gebildet. Die Auslenkung der Drähte bei Axial- und Torsionsbeanspruchung ist dabei nicht für alle Längenbereiche der Drähte und für alle Anordnungen der Drähte im Netz einheitlich.

In einer anderen Ausführung ist das Flächengebilde aus radial speichenförmig gespannten Drähten gebildet. Diese Ausführung ergibt eine im Wesentlichen gleiche Verteilung der Dehnungsbeanspruchung über die gesamte Länge der Drähte bei einer axialen Auslenkung. Ebenso werden sämtliche Drähte bei einer Torsionsbeanspruchung in gleicher Weise ausgelenkt und wirken in gleicher Weise für die Torsionssteifigkeit zusammen.

In einer Weiterbildung der Erfindung werden die Flächengebilde, die im Wesentlichen die axiale Abfederung der Bandscheibenprothese bewirken, am Außenumfang durch elastische Elemente umschlossen, die die einander zugewandten Außenränder der Endplatten miteinander verbinden. Diese elastischen Elemente können als koaxiale Schraubenfedern ausgebildet sein oder als über den Umfang verteilte mäanderförmige Federelemente. Diese elastischen Elemente führen zu einer zusätzlichen Abstützung bei Flexion und Rotation der Bandscheibenprothese, d. h. bei einem gegenseitigen Verkippen der Ebenen der Endplatten und bei einer gegenseitigen Rotation der Endplatten.

Im Folgenden wird die Erfindung anhand von in der Zeichnung dargestellten Ausführungsbeispielen näher erläutert. Es zeigen
- Fig. 1: eine perspektivische Ansicht einer Bandscheibenprothese in einer ersten Ausführung,

- Fig. 2: einen Axialschnitt durch die Bandscheibenprothese der Figur 1,
- Fig. 3: eine Seitenansicht einer Bandscheibenprothese in einer zweiten Ausführung,
- Fig. 4: einen Axialschnitt durch die Bandscheibenprothese der Figur 3 und
- Fig. 5: einen Schnitt durch die Bandscheibenprothese der Figur 3 gemäß der Schnittlinie A-A in Figur 3.

In der Zeichnung ist die implantierbare Bandscheibenprothese nur schematisch dargestellt. Detailliertere Anpassungen in der Querschnittsform und in der Ausbildung der Endplatten an die anatomischen Gegebenheiten der Implantation sind nicht gezeigt, da diese für die Erfindung nicht wesentlich sind.

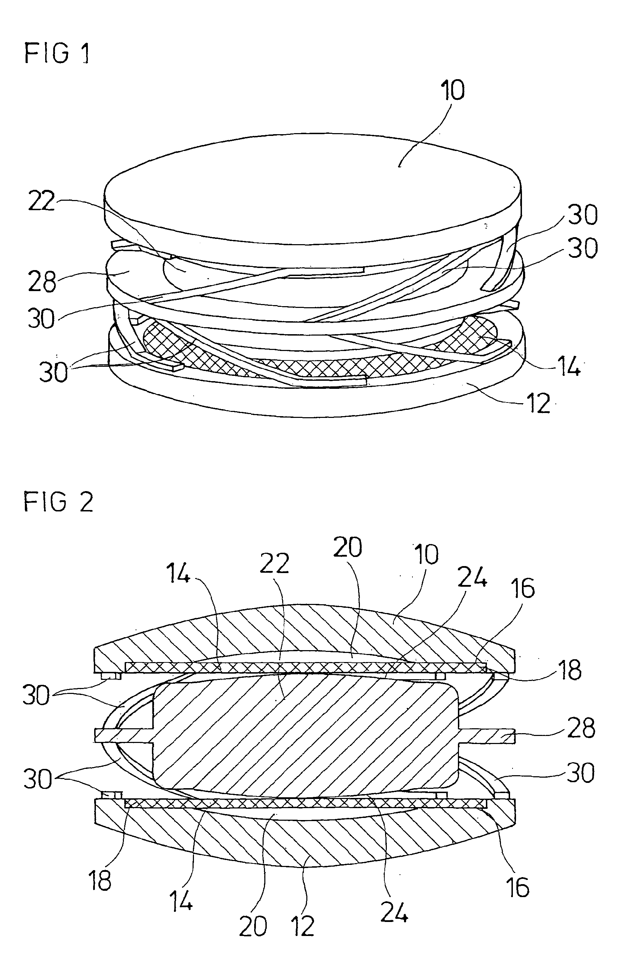

In dem Ausführungsbeispiel der Figuren 1 und 2 weist die implantierbare Bandscheibenprothese eine obere kraniale Endplatte 10 und eine untere kaudale Endplatte 12 auf. In der Zeichnung sind die Endplatten 10 und 12 kreisrund dargestellt. Eine Anpassung der Außenkontur und der jeweiligen Auswölbung der Endplatten 10 und 12 an die Form der Wirbelkörper ist selbstverständlich möglich. Die Endplatten 10 und 12 sind aus einem biokompatiblen formstabilen Werkstoff hergestellt, z. B. aus Metall oder Kunststoff.

An den einander zugewandter Flächen der Endplatten 10 und 12 ist jeweils ein Flächengebilde 14 angebracht, welches aus Drähten einer Memory-Metalllegierung, z.B. aus einer Nickel-Titan-Legierung besteht, die bei der menschlichen Körpertemperatur superelastische Eigenschaften aufweist. In dem Ausführungsbeispiel der Figuren 1 und 2 sind die Drähte zur Bildung des Flächengebildes 14 sich kreuzend netzartig gespannt, wie dies z.B. bei den Saiten eines Tennisschlägers der Fall ist. Die Flächengebilde 14 der beiden Endplatten 10 und 12 sind identisch ausgebildet und weisen die Form einer Kreisscheibe auf. Die Flächengebilde 14 sind jeweils in eine kreisförmige Vertiefung 16 der einander zugewandten Flächen der Endplatten 10 bzw. 12 eingesetzt, wobei der Innenumfang der Vertiefung 16 dem Außenumfang des Flächengebildes 14 entspricht. In ihrem äußeren Umfangsbereich 18 sind die Flächengebilde 14 mit der jeweiligen Endplatte 10 bzw. 12 fest verbunden. Die Vertiefungen 16 sind gegen ihr Zentrum hin mit einer konzentrischen konkaven Einwölbung 20 ausgebildet, die somit von dem Flächengebilde 14 mit einem gegen das Zentrum hin zunehmenden axialen Abstand überspannt wird.

Zwischen die Flächengebilde 14 der beiden Endplatten 10 und 12 ist koaxial ein Kern 22 aus einem formstabilen biokompatiblen Werkstoff, vorzugsweise aus Kunststoff eingesetzt. Der Kern 22 hat im Wesentlichen die Form eines flachen zu den Endplatten 10 und 12 koaxialen Kreiszylinders. Der Durchmesser des Kerns 22 ist etwas geringer als der Durchmesser der Flächengebilde 14. Die axialen Stirnflächen des Kerns 22 sind mit einer konzentrischen konvexen Auswölbung 24 ausgebildet, die in etwa komplementär zu den Einwölbungen 20 der Endplatten 10 bzw. 12 geformt ist. Mit ihrer axial am stärksten ausgewölbten Mitte der Stirnflächen sitzt der Kern 22 auf dem zentrischen Mittenbereich 26 der Flächengebilde 14 auf und ist vorzugsweise in diesem Mittenbereich 26 mit dem Flächengebilde 14 fest verbunden. Insbesondere kann eine kraftschlüssige Verbindung dadurch hergestellt werden, dass der Kern 22 mit dem Mittenbereich 26 des Flächengebildes 14 verklebt oder verschweißt ist oder dass in den Mittenbereich 26 ein Ring eingesetzt ist, der auf einen axialen Zapfen des Kerns 22 aufgepresst wird.

An dem Außenumfang des Kerns 22 ist in dessen axialer Mitte ein nach außen abstehender kreisringförmiger Flansch 28 angeformt. Der Außenumfang des Flansches 28 entspricht im Wesentlichen dem Außenumfang der Endplatten 10 und 12. Zwischen den Flansch 28 und die kraniale Endplatte 10 einerseits und zwischen den Flansch 28 und die kaudale Endplatte 12 andererseits sind jeweils elastische Elemente eingesetzt, die im Ausführungsbeispiel der Figuren 1 und 2 als Schraubenfedern 30 ausgebildet sind. Die Schraubenfedern 30 zwischen dem Flansch 28 und der kranialen Endplatte 10 und die Schraubenfedern 30 zwischen dem Flansch 28 und der kaudalen Endplatte 12 sind dabei als gegenläufige Schraubenfedern ausgebildet.

Wird die in die Wirbelsäule implantierte Bandscheibenprothese axial auf Druck beansprucht, werden die Endplatten 10 und 12 axial zusammengedrückt. Dabei drückt der Kern 22 die gespannten Flächengebilde 14 jeweils mit ihrem Mittenbereich 26 in die Einwölbungen 20 der Endplatten 10 und 12. Da die Flächengebilde 14 in ihrem Umfangsbereich 18 an den Endplatten 10 bzw. 12 festgelegt sind, führt diese Wölbung des Mittenbereichs 26 der Flächengebilde 14 zu einer Dehnung der Drähte des Flächengebildes 14. Da die axiale Auslenkung des Mittenbereichs 26 dabei klein ist im Vergleich zu den radialen Abmessungen der Flächengebilde 14, führt diese Auswölbung im Wesentlichen zu einer Dehnung der Drähte und nur zu einer sehr geringen Biegung. Die Auswölbung 24 der Stirnflächen des Kernes 22 bewirkt dabei, dass sich die Drähte bei zunehmendem Eindringen des Kernes 22 in die Einwölbungen 20 an die flache Krümmung der Auswölbung 24 anlegen, so dass auch hierdurch zusätzlich eine stärkere Biegung der Drähte ausgeschlossen ist. Da die Drähte des Flächengebildes 14 somit im Wesentlichen axial gedehnt und nur minimal gebogen werden, ergibt sich eine hohe axiale Rückstellkraft der Flächengebilde 14 bei einer minimalen Dehnungsbeanspruchung des Materials der Drähte. Diese geringe Dehnungsbeanspruchung bedeutet eine hohe Dauerstandfestigkeit. Die flache scheibenförmige Ausbildung der Flächengebilde 14 beansprucht nur eine geringe axiale Bauhöhe, so dass die gesamte axiale Abmessung der Bandscheibenprothese an die anatomischen Anforderungen angepasst werden kann, ohne dass hierbei Einschränkungen durch die Abfederung mittels der Flächengebilde 14 berücksichtigt werden müssen.

Aufgrund der festen Verbindung des Umfangbereiches 18 der Flächengebilde 14 mit den Endplatten 10 bzw. 12 und des Mittenbereiches 26 mit dem Kern 22 federn die Flächengebilde 14 auch Torsionsbewegungen der Endplatten 10 und 12 gegeneinander ab.

Die Schraubenfedern 30 bewirken zum einen eine mechanische Verbindung zwischen den Endplatten 10 und 12. Außerdem bewirken die Schraubenfedern 30 eine gegenseitige Abstützung der Endplatten 10 und 12 gegen ein Verkippen der Ebenen dieser Endplatten 10 und 12 bei einer Biegung der Wirbelsäule. Schließlich stützen die Schraubenfedern 30 auf die Endplatten 10 und 12 einwirkende Torsionskräfte ab, wobei die gegenläufige Ausbildung der Schraubenfedern 30 oberhalb und unterhalb des Flansches 28 eine symmetrische Abstützung gegen rechts-und linksdrehende Torsionskräfte gewährleistet.

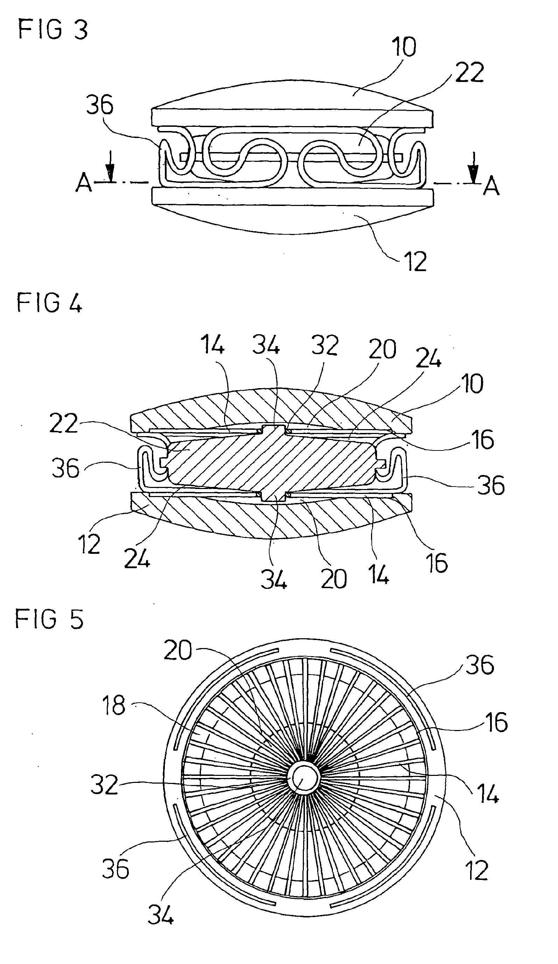

Ein weiteres Ausführungsbeispiel der Bandscheibenprothese ist in den Figuren 3 bis 5 dargestellt. Soweit diese Bandscheibenprothese mit dem Ausführungsbeispiel der Figuren 1 und 2 übereinstimmt, sind dieselben Bezugszeichen verwendet und auf die vorangehende Beschreibung wird Bezug genommen.

Das Ausführungsbeispiel der Figuren 3 bis 5 unterscheidet sich von dem Ausführungsbeispiel der Figuren 1 und 2 im Wesentlichen durch die Ausgestaltung der Flächengebilde 14. In dem Ausführungsbeispiel der Figuren 3 bis 5 bestehen die Flächengebilde 14 jeweils aus Drähten einer Memory-Metalllegierung, die radial speichenförmig gespannt sind. Im Umfangsbereich 18 sind die Drähte an der jeweiligen Endplatte 10 bzw. 12 festgelegt, während die radial inneren Enden der Drähte im Mittenbereich 26 an einem Ring 32 befestigt sind, der zentrisch an der jeweiligen ausgewölbten Stirnfläche des Kerns 22 vorzugsweise kraftschlüssig festgelegt ist. In dem dargestellten Ausführungsbeispiel kann dieser Ring 32 hierzu auf einen koaxialen mittigen Zapfen 34 aufgepresst sein, der an den Stirnflächen des Kernes 22 angeformt ist.

Aufgrund der radialen Anordnung der Drähte des Flächengebildes 14 werden in dieser Ausführung die Drähte bei einer axialen Auslenkung der Flächengebilde 14 nahezu ausschließlich in Längsrichtung gedehnt, während die Krümmung aufgrund des Verhältnisses von axialer Auslenkung zu radialer Abmessung der Flächengebilde 14 minimal ist. Auch eine Torsion der Endplatten 10 und 12 führt im Wesentlichen zu einer Biegung der radialen Drähte und entsprechend im Wesentlichen zu einer Dehnung der Drähte in Längsrichtung.

Auch für die Abstützung der Endplatten 10 und 12 an ihrem Außenumfang ist in dem Ausführungsbeispiel der Figuren 3 bis 5 eine Alternative zu den Schraubenfedern 30 und dem Flansch 28 des Ausführungsbeispieles der Figuren 1 und 2 gezeigt. Zwischen die über den Kern 22 und die Flächengebilde 14 radial überstehenden Ränder der Endplatten 10 und 12 ist in dem Ausführungsbeispiel der Figuren 3 bis 5 als elastisches Element ein mäanderförmig gebogener in Umfangsrichtung verlaufender Federdraht 36 eingesetzt. Die in Umfangsrichtung jeweils aufeinanderfolgenden Biegungen des Federdrahtes 36 haben dabei jeweils die Form eines liegenden S, welches spiegelsymmetrisch zu dem in Umfangsrichtung anschließenden S ist, wie in Figur 3 zu sehen ist. Der mäanderförmig gebogene Federdraht 36 ergibt auch bei dieser Ausführung eine Abstützung der Endplatten 10 und 12 gegen ein Verkippen ihrer jeweiligen Ebenen, d. h. gegen eine Flexion der Wirbelsäule. Außerdem stützt der Federdraht 36 die Endplatten 10 und 12 gegen Torsionskräfte ab, wobei die Aufeinanderfolge von spiegelsymmetrischen S-förmigen Mäanderwindungen eine symmetrische Abstützung gegen rechts-und linksdrehende Torsionskräfte bewirkt.

Es ist ohne Weiteres ersichtlich, dass die Federstrukturen zwischen den Endplatten 10 und 12 am Außenumfang durch geeignete Materialien abgedeckt und umschlossen werden können. Eine solche Ummantelung ist in der Zeichnung nicht dargestellt, da sie für die erfindungsgemäße Abfederung der Bandscheibenprothese nicht wesentlich ist.

Die gesamte Dimensionierung der Bandscheibenprothese kann den anatomischen Gegebenheiten der Implantationssituation angepasst werden. Repräsentative Abmessungen sind dabei ein Durchmesser von etwa 28 mm und eine axiale Höhe von etwa 10 mm. Die Endplatten 10 und 12 können dabei mit einer axialen Randhöhe am Außenumfang von etwa 1 mm ausgebildet sein. Dabei ergibt sich ein axialer lichter Abstand der Endplatten 10 und 12 von etwa 8 mm. Der axiale Federweg der Endplatten 10 und 12 kann dabei bis etwa maximal 1,5 mm betragen. Die axiale Rückstellkraft der Flächengebilde 14 steigt dabei von etwa 1000 N bei einem axialen Hub von 0,7 mm bis zu etwa 2000 N bei einem axialen Hubweg von 1,5 mm an.

### Bezugszeichenliste

- 10: kraniale Endplatte
- 12: kaudale Endplatte
- 14: Flächengebilde
- 16: Vertiefung
- 18: Umfangsbereich
- 20: Einwölbung
- 22: Kern
- 24: Auswölbung
- 26: Mittenbereich
- 28: Flansch
- 30: Schraubenfedern
- 32: Ring
- 34: Zapfen
- 36: Federdraht

## Patentansprüche

1. Bandscheibenprothese mit einer kranialen Endplatte (10), mit einer axial beabstandeten kaudalen Endplatte (12) und mit zwischen diesen Endplatten (10, 12) angeordneten Federmitteln, welche diese Endplatten (10, 12) druckelastisch gegeneinander abstützen und aus einer Memory-Metalllegierung bestehen, die bei Körpertemperatur superelastische Eigenschaften aufweist,
**dadurch gekennzeichnet, dass** die Federmittel wenigsten ein zu den Endplatten (10, 12) im Wesentlichen flächenparalleles Flächengebilde (14) aufweisen, welches aus in der Ebene des Flächengebildes (14) gespannten Drähten aus der Memory-Metalllegierung besteht, dass das Flächengebilde (14) mit seinem zentrischen Mittenbereich (26) mit der einen Endplatte (12) und mit seinem radial von dem Mittenbereich (26) beabstandeten äußeren Umfangsbereich (18) mit der anderen Endplatte (10) verbunden ist und dass der Mittenbereich (26) und der Umfangsbereich (18) des Flächengebildes (14) in axialer Richtung gegeneinander auslenkbar angeordnet sind.

2. Bandscheibenprothese nach Anspruch 1,
**dadurch gekennzeichnet, dass** mit jeder Endplatte (10,1 12) jeweils ein Flächengebilde (14) verbunden ist und dass ein zu den Flächengebilden (14) im Wesentlichen koaxialer formstabiler Kern (22) die Flächengebilde (14) verbindet.

3. Bandscheibenprothese nach Anspruch 2,
**dadurch gekennzeichnet, dass** die Flächengebilde (14) jeweils mit ihrem Umfangsbereich (18) mit der zugehörigen Endplatte (10, 12) und mit ihrem Mittenbereich (26) mit dem Kern (22) verbunden, vorzugsweise kraftschlüssig verbunden sind.

4. Bandscheibenprothese nach einem der Ansprüche 1 bis 3,
**dadurch gekennzeichnet, dass** die mit dem Umfangsbereich (18) des Flächengebildes (14) verbundenen Endplatten (10, 12) eine konzentrische konkave Einwölbung (20) aufweisen, in welche der Mittenbereich (26) des Flächengebildes (14) axial eingedrückt werden kann.

5. Bandscheibenprothese nach einem der Ansprüche 1 bis 4,
**dadurch gekennzeichnet, dass** die mit dem Mittenbereich (26) des Flächengebildes (14) verbundene Endplatte bzw. der mit dem Mittenbereich (26) verbundene Kern (22) eine konzentrische konvexe Auswölbung (24) aufweisen, an welche sich das Flächengebilde (14) bei axialer Druckbelastung anlegt.

6. Bandscheibenprothese nach einem der Ansprüche 1 bis 5,
**dadurch gekennzeichnet, dass** das Flächengebilde (14) aus sich netzartig kreuzend gespannten Drähten gebildet ist.

7. Bandscheibenprothese nach einem der Ansprüche 1 bis 5,
**dadurch gekennzeichnet, dass** das Flächengebilde (14) aus radial speichenförmig gespannten Drähten gebildet ist.

8. Bandscheibenprothese nach einem der vorhergehenden Ansprüche,
**dadurch gekennzeichnet, dass** die einander zugewandten Außenränder der Endplatten (10, 12) durch elastische Elemente miteinander verbunden sind, die die Federmittel koaxial umschließen.

9. Bandscheibenprothese nach Anspruch 8,
**dadurch gekennzeichnet, dass** die elastischen Elemente als zu den Endplatten (10, 12) koaxiale Schraubenfedern (30) ausgebildet sind.

10. Bandscheibenprothese nach Anspruch 9,
**dadurch gekennzeichnet, dass** gegenläufige Schraubenfedern (30) vorgesehen sind.

11. Bandscheibenprothese nach Anspruch 8,
**dadurch gekennzeichnet, dass** die elastischen Elemente durch einen in Umfangsrichtung verlaufend mäanderförmig gebogenen Federdraht (36) gebildet sind.
